# EUROPEAN PATENT SPECIFICATION

(11) **EP 4 261 274 B1**
(45) Date of publication and mention of the grant of the patent: **19.03.2025**
(21) Application number: 23167274.2
(22) Date of filing: 11.04.2023
(51) Int. Cl.: C12N 5/00, C12M 3/00

(54) **MEROXAPOLS FOR CELL CULTURE**
MEROXAPOLEN FÜR ZELLKULTUR
MEROXAPOLS POUR CULTURE CELLULAIRE

(30) Priority: 13.04.2022 EP 22168244
(43) Date of publication of application: 18.10.2023
(73) Proprietor: Merck Patent GmbH, 64293 Darmstadt (DE)
(72) Inventor: Antonello, Alice, 64283 Darmstadt (DE); Hofmann, Tim, 64293 Darmstadt (DE); Rapp, Almut, 64293 Darmstadt (DE)
(74) Representative: Merck Patent Association

(56) References cited:
- WO-A2-2004/045645
- CHANG DAVID ET AL: "Investigation of interfacial properties of pure and mixed poloxamers for surfactant-mediated shear protection of mammalian cells", COLLOIDS AND SURFACES B: BIOINTERFACES, ELSEVIER AMSTERDAM, NL, vol. 156, 16 May 2017 (2017-05-16), pages 358 - 365, XP085082047, ISSN: 0927-7765, DOI: 10.1016/J.COLSURFB.2017.05.040
- HAOFAN PENG ET AL: "Mechanism investigation for poloxamer 188 raw material variation in cell culture", BIOTECHNOLOGY PROGRESS, vol. 32, no. 3, 1 May 2016 (2016-05-01), Hoboken, USA, pages 767 - 775, XP055545565, ISSN: 8756-7938, DOI: 10.1002/btpr.2268
- ALEXANDRIDIS ET AL: "Poly(ethylene oxide)/poly(propylene oxide) block copolymer surfactants", CURRENT OPINION IN COLLOID & INTERFACE SCIENCE, LONDON, GB, vol. 2, no. 5, 1 October 1997 (1997-10-01), pages 478 - 489, XP027132127, ISSN: 1359-0294, [retrieved on 19971001]
- SCHMOLKA I R: "A REVIEW OF BLOCK POLYMER SURFACTANTS", JOURNAL OF THE AMERICAN OIL CHEMISTS SOCIETY, SPRINGER, DE, vol. 54, no. 3, 1 January 1977 (1977-01-01), pages 110 - 116, XP009037148, ISSN: 0003-021X, DOI: 10.1007/BF02894385

## Description

The present invention relates to the use of meroxapols, also called "reverse poloxamers", as cell culture media additives. The meroxapols are suitable for foam reduction as well as for shear stress protection.

### Background of the invention

Poloxamers, especially Poloxamer 188, are used in many industrial applications, cosmetics, and pharmaceuticals. They are also used in cell culture media processes. The addition of poloxamers, especially poloxamer 188, to cell culture media improves cell viability significantly. High cell viability is crucial for optimal protein production. Why poloxamers improve cell viability is not fully understood. It is believed that poloxamers reduce shear stress and in this way protect the cells from damage. Poloxamer, being a nonionic surfactant, is likely to concentrate at the gas bubble/medium interface and could prevent cell attachment to gas bubbles and in this way prevent cells from damage when gas bubbles burst. It may also reduce shock when bubbles burst. Some publications claim that poloxamers improve the oxygen transfer rate from the gas into the liquid phase, but other publications contradict these findings. There are also indications that poloxamers may "repair" small defects in cell membranes.

Unfortunately, Poloxamer 188 contributes significantly to stabilization of the foam produced in sparged bioreactors. Therefore, typically, antifoam agents are used in addition to poloxamers. Examples of antifoam agents are preferably agents comprising polydimethylsiloxane (PDMS) as well as optionally silica particles, also called e.g., dimethicone or simethicone.

The addition of an antifoam agent does not solve all the issues, because it is generally added manually and, if the foam level is not constantly monitored, the risk of foam overflow persists. The silica-based antifoams get deactivated during their action because of the silica segregation. In addition, the antifoam agents cannot be added in great amount because they may be toxic at higher concentrations. Simethicone is silicon oil based and is not filterable on PES membranes, therefore it cannot be sterilized with PES filtration, that is why antifoam C cannot be added directly in the cell culture media formulation. Foam issues become even worse in the perspective of higher cell density cultivations because more oxygen is required which leads to more sparging and thus more foam.

Therefore, a non- or low-foaming alternative to Poloxamer 188 would be desirable.

Schmolka I. R. (Schmolka, Journal of the American Oil Chemists' Society (1977), 54(3), 110-16) and Murhammer (D. W. Murhammer, C. F. Goochee, Biotechnol. Prog. 1990, 6, 142-148) published data about the properties of other poloxamers beside poloxamer 188. They also discussed the properties of meroxapols. Albeit they found some candidates that show promising features concerning shear stress protection and foam stabilization, they could not identify ideal candidates. Some were toxic for the insect cells and most candidates still required the addition of an antifoam agent.

Consequently, there is still the need to find an alternative to poloxamer 188 which does not only provide shear stress protection but on the other hand also reduces foam formation and is non-toxic for the cells.

It has been found that certain meroxapols fulfill all requirements concerning toxicity, foam formation, and shear stress protection. The requirements for an ideal suitability in cell culture are a certain peak molecular weight combined with a defined percentage of the PEO (polyethylene oxide) part. This results in comparable shear stress protection to poloxamer 188, i.e., similar viable cell density, viability and IgG productivity, and, at the same time, eliminates the need to add antifoam agent. Employing these meroxapols, no or only little foam is formed during the process, which makes the addition of an antifoam agent unnecessary.

The present invention is thus directed to a cell culture medium comprising a meroxapol with a peak molecular weight Mₚ between 1000 and 8000 g/mol, preferably between 1000 and 5000 g/mol and a polyethylene oxide percentage (%EO) between 55 and 95% (w/w).

In a preferred embodiment, the peak molecular weight Mₚ is between 1800 and 3500 g/mol.

In another preferred embodiment, the polyethylene oxide percentage is between 55 and 80%.

In a preferred embodiment, the cell culture medium comprises the meroxapol in an amount between 0.1 to 10 g/L calculated for the liquid medium. In a very preferred embodiment, the amount of meroxapol is between 0.5 to 5 g/L calculated for the liquid medium.

In one embodiment the cell culture medium is a chemically defined medium.

In one embodiment the cell culture medium is a dry powder or a dry compacted medium.

In another embodiment, the cell culture medium comprises at least one or more saccharide components, one or more amino acids, one or more vitamins or vitamin precursors, one or more salts, one or more buffer components, one or more co-factors and one or more nucleic acid components.

In a preferred embodiment the cell culture medium does not comprise any antifoam agent.

The present invention is also directed to a process for culturing cells whereby the cells are cultured in a liquid medium comprising a meroxapol with a peak molecular weight Mₚ between 1000 and 8000 g/mol, preferably between 1000 and 8000 g/mol, and a polyethylene oxide percentage between 55 and 95% (w/w). Preferably the process includes agitation and/or sparging.

In a preferred embodiment, the peak molecular weight Mₚ is between 1800 and 3500 g/mol.

In another preferred embodiment, the polyethylene oxide percentage is between 55 and 80%.

In a preferred embodiment, the amount of antifoam agent in the liquid medium is reduced compared to a cell culture medium that is otherwise identical but comprises poloxamer 188 instead of a meroxapol as defined above, very preferred the medium does not comprise any antifoam agent.

The present invention is also directed to a method for reducing foam formation in agitated and/or sparged cell cultures whereby the cells are cultured in a liquid medium comprising a meroxapol with a peak molecular weight Mₚ between 1000 and 8000 g/mol and a polyethylene oxide percentage between 55 and 95% (w/w) and the foam formation is reduced compared to cells cultured under the same conditions in a cell culture medium that comprises poloxamer 188 instead of a meroxapol with a peak molecular weight Mₚ between 1000 and 8000 g/mol and a polyethylene oxide percentage between 55 and 95% (w/w). Preferably the medium does not comprise any antifoam agent.

### Figures

Figure 1 shows the foam evolution during continuous air sparging at 37 °C for selected meroxapols. The meroxapol solutions are prepared in cell culture media and have concentration 1000 ppm. The curves reported are the output of one measurement, selected out of the replicates as the most representative of the trend for each poloxamer. Details can be found in Example 1.
Figure 2 shows the foam evolution during continuous air sparging at 37 °C for selected meroxapols. The meroxapol solutions are prepared in cell culture media and have concentration 2000 ppm. The curves reported are the output of one measurement, selected out of the replicates as the most representative of the trend for each poloxamer. Details can be found in Example 1.
Figure 3 shows the average maximum foam height referred to each meroxapol solution in cell culture media. The measurements were performed at 37 °C. The values are the average of the replicates. Light grey bars, solution concentration 1000 ppm; dark grey bar, solution concentration 2000 ppm. Details can be found in Example 1.
Figure 4 shows the average plateau height referred to each meroxapol solution in cell culture media. The measurements were performed at 37 °C. The values are the average of the replicates. Light grey bars, solution concentration 1000 ppm; dark grey bar, solution concentration 2000 ppm. Details can be found in Example 1.
Figure 5 shows the viability (%) of CHOK1 cells during the fed-batch in spin tubes. Each value is the average of the 4 replicates of each condition.
Figure 6 shows the viable cell density (VCD) of CHOK1 cells during the fed-batch in spin tubes. Each value is the average of the 4 replicates of each condition.
Figure 7 shows IgG productivity of CHOK1 cells during the fed-batch in spin tubes. Each value is the average of the 4 replicates of each condition.
Figure 8 shows the viability (%) of CHOK1 cells during the fed-batch culture in spin tubes to demonstrate the necessity of adding poloxamers and/or meroxapols . Each value is the average of the 4 replicates of each condition.
Figure 9 shows the viability (%) of CHOK1 cells during the fed-batch in bioreactors. Each data point is the average of three replicates of each condition. Error bars represent the standard deviation of the average.
Figure 10 shows the viable cell density (VCD) of CHOK1 cells during the fed-batch in bioreactor. Each data point is the average of the three replicates of each condition.
Figure 11 shows IgG productivity of CHOK1 cells during the fed-batch in bioreactor. Each data point is the average of the three replicates of each condition. Error bars represent the standard deviation of the average. For certain data points, the error bars are not visible because the error is too little.

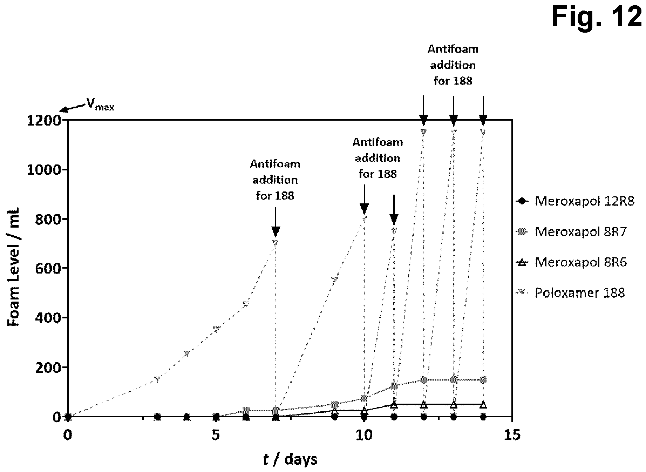

Figure 12 shows foam evolution during fed-batch cultivation in bioreactor. The data points refer to the foam level of one bioreactor per sample selected as representative. The only cultivations that required antifoam C addition to avoid foam overflow were the ones supported by Poloxamer 188. The other meroxapols stabilized only a very little amount of foam, no risk of overflow and no need of antifoam C.
   Details for Figures 5 to 12 can be found in Example 2. In Figures 5, 6, 7, 10, 11 and 12 error bars represent the standard deviation of the average. The data points referring to Poloxamer 188 are connected by dashed lines to better distinguish them from the rest of the samples.
Figure 13 shows the molecular weight distribution determined with size exclusion chromatography (see Method 1) for meroxapol 8R6. Mp indicates the peak molecular weight of the specific meroxapol.

### Definitions

Before describing the present invention in detail, it is to be understood that this invention is not limited to specific compositions or process steps, as such may vary. It must be noted that, as used in this specification and the appended claims, the singular form "a", "an" and "the" include plural referents unless the context clearly dictates otherwise. Thus, for example, reference to "a poloxamer" includes a plurality of poloxamers and the like. Unless defined otherwise, all technical and scientific terms used herein have the same meaning as commonly understood by one of ordinary skill in the art to which this invention is related. The following terms are defined for purposes of the invention as described herein.

The term "bioreactor," as used herein, refers to any manufactured or engineered device or system that supports a biologically active environment. In some instances, a bioreactor is a vessel or tank in which a cell culture process is carried out which involves organisms or biochemically active substances derived from such organisms. Such a process may be either aerobic or anaerobic. Commonly used bioreactors are typically cylindrical, ranging in size from liters to cubic meters, and are often made of stainless steel. In some embodiments described herein, a bioreactor might contain a disposable constituent made of a material other than steel and is disposable. In some embodiments that is a disposable bag where in the biologically active environment is maintained. It is contemplated that the total volume of a bioreactor may be any volume ranging from 100 mL to up to 10,000 Liters or more, depending on a particular process.

An agitated cell culture is a cell culture that is performed in a bioreactor by permanently or one or several times during the cell culture agitating the cell culture medium with the cells in the bioreactor.

Agitation can for example be done by stirring, rocking, or shaking. In stirred tank bioreactors, one or several impellers may be deployed, depending on the geometry of the vessels. Stirrer types will be selected according to the main mixing task and requirements of the process.

A sparged cell culture is a cell culture in which a gas is introduced into the bioreactor. This is typically done with a sparger, also called bubbler or aerator.

Typically, in a sparged cell culture, a mixture of air, oxygen, carbon dioxide, and nitrogen is introduced into the bioreactor with a sparger. Typical spargers for cell culture processes include drilled-hole ring spargers, sintered micro-spargers, open pipe spargers, and hybrid forms. Surface aeration also contributes to oxygen transfer, but the impact of surface aeration decreases during scale-up. Both stirrer and sparger types in mammalian cell culture processes will often be selected according to the cells' shear sensitivity (Nienow, A.W., 2006. Reactor engineering in large scale animal cell culture, Cytotechnology, 50(1-3), p.9).

A cell culture medium according to the present invention is any mixture of components which maintains and/or supports the in vitro growth of cells and/or supports a particular physiological state, typically by providing at least one nutrient to the cells. It might be a complex medium or a chemically defined medium. The cell culture medium can comprise all components necessary to maintain and/or support the in vitro growth of cells or only some components so that further components are added separately. Examples of cell culture media according to the present invention are full media which comprise all components necessary to maintain and/or support the in vitro growth of cells as well as media supplements or feeds. In a preferred embodiment, the cell culture medium is a full medium, a perfusion medium or a feed medium. A full medium also called basal medium typically has a pH between 6.7 and 7.8. A feed medium preferably has a pH below 8.5.

Typically, the cell culture media according to the invention are used to maintain and/or support the growth of cells in a bioreactor.

A feed or feed medium is a cell culture medium that is not the basal medium that supports initial growth and production in cell culture but the medium which is added at a later stage to prevent depletion of nutrients and sustains the production phase. A feed medium can have higher concentrations of some components compared to a basal culture medium. For example, some components, such as, for example, nutrients including amino acids or carbohydrates, may be present in the feed medium at about 5X, 6X, 7X, 8X, 9X, 10X, 12X, 14X, 16X, 20X, 30X, 50X, 100x, 200X, 400X, 600X, 800X, or even about 1000X of the concentrations in a basal medium.

A mammalian cell culture medium is a mixture of components which maintain and/or support the in vitro growth of mammalian cells. Examples of mammalian cells are human or animal cells, preferably CHO cells, COS cells, I VERO cells, BHK cells, AK-1 cells, SP2/0 cells, L5.1 cells, hybridoma cells, or human cells.

Chemically defined cell culture media are cell culture media that do not comprise any chemically undefined substances. This means that the chemical composition of all the chemicals used in the media is known. The chemically defined media do not comprise any yeast, animal, or plant tissues; they do not comprise feeder cells, serum, hydrolysates, extracts or digests or other poorly defined components. Chemically undefined or poorly defined chemical components are those whose chemical composition and structure are not known, are present in varying composition or could only be defined with enormous experimental effort - comparable to the evaluation of the chemical composition and structure of a protein like insulin, albumin or casein.

A powdered cell culture medium or a dry powder medium is a cell culture medium typically resulting from a milling process or a lyophilization process. That means the powdered cell culture medium is a granular, particulate medium - not a liquid medium. The term "dry powder" may be used interchangeably with the term "powder;" however, "dry powder" as used herein simply refers to the gross appearance of the granulated material and is not intended to mean that the material is completely free of complexed or agglomerated solvent unless otherwise indicated.

A dry granulated medium is a dry medium resulting from a wet or dry granulation process, e.g. by spray drying, wet granulation, or dry compaction, typically have particle sizes above 0.5 mm, e.g. between 0.5 and 5 mm. Dry compaction is typically done in a roll press. US 6,383,810 B2 discloses a method of producing a wet granulated eukaryotic cell culture medium powder. The method comprises wetting a dry powder cell culture medium with a solvent and then re-drying the moistened medium to obtain a dry granulated cell culture medium. Preferably, the dry granulated medium is a medium resulting from roller compaction of a dry powder medium. The term dry as used herein simply refers to the gross appearance of the granulated material and is not intended to mean that the material is completely free of complexed or agglomerated solvent unless otherwise indicated.

For use in cell culture, i.e., for culturing the cells, a liquid cell culture medium is added to the cells. A dry powder or dry compacted cell culture medium is thus dissolved in a suitable amount of liquid like water or an aqueous buffer, to generate a liquid cell culture medium that can be contacted with the cells. As the composition of the liquid cell culture medium is what directly influences the cells, the concentrations of cell culture media are often provided in weight per liter, like mg per liter, defining the concentration of a component in the liquid medium to be added to the cells. The amount of the ingredients of a dry powder or dry compacted medium need to be adjusted such that when dissolved in a certain amount of liquid the aimed concentration of the components in the resulting liquid medium is reached.

Cells to be cultured with the media according to the present invention may be prokaryotic cells like bacterial cells or eukaryotic cells like plant or animal cells. The cells can be normal cells, immortalized cells, diseased cells, transformed cells, mutant cells, somatic cells, germ cells, stem cells, precursor cells, or embryonic cells, any of which may be established or transformed cell lines or obtained from natural sources.

Average molecular weight is determined by SEC as follows:
weight average molecular weight: M_{w} = Σᵢ Nᵢ Mᵢ² / (Σᵢ Nᵢ Mᵢ)
number average molecular weight: Mₙ = Σᵢ Nᵢ Mᵢ / (Σᵢ Nᵢ)
peak molecular weight: Mₚ = molecular weight at maximum Nᵢ
Nᵢ = number of polymer species in fraction i
Mᵢ = molecular weight of polymer species in fraction i
SEC conditions:
   Calibration standards: PEG (details see Examples, Method 1)
   Eluent: THF
   Flow rate: 1 ml/min
   Injection volume: 100 µl
   Column: particle size = 5 µm, material = styrene-divinylbenzene
   Temperature: 40 °C

Cell culture media can be in the form of aqueous liquids or in the form of dry powders which for use are dissolved in water or an aqueous buffer.

A person skilled in the art is able to choose a suitable cell culture medium for the specific envisaged purpose.

The cell culture media, especially the full media which comprises all components necessary to maintain and/or support the *in vitro* growth of cells, according to the present invention typically comprise at least one or more saccharide components, one or more amino acids, one or more vitamins or vitamin precursors, one or more salts, one or more buffer components, one or more co-factors, and one or more nucleic acid components. They may also additionally comprise chemically defined biochemicals such as recombinant proteins, e.g., rinsulin, rBSA, rTransferrin, rCytokines etc.

Saccharide components are all mono- or di-saccharides, like glucose, galactose, ribose, or fructose (examples of monosaccharides) or sucrose, lactose or maltose (examples of disaccharides).

Examples of amino acids according to the invention are tyrosine, the proteinogenic amino acids, especially the essential amino acids, leucine, isoleucine, lysine, methionine, phenylalanine, arginine, threonine, tryptophane, and valine, as well as the non-proteinogenic amino acids like D-amino acids, whereby the L-amino acids are preferred.

The term of the amino acids further includes the salts of the amino acids, like the sodium salts, or the respective hydrates or hydrochlorides.

For example, tyrosine means L- or D- tyrosine, preferably L-tyrosine, as well as its salts or hydrates or hydrochlorides.

Examples of vitamins are Vitamin A (Retinol, retinal, various retinoids, and four carotenoids), Vitamin B₁ (Thiamine), Vitamin B₂ (Riboflavin), Vitamin B₃ (Niacin, niacinamide), Vitamin B₅ (Pantothenic acid), Vitamin B₆ (Pyridoxine, pyridoxamine, pyridoxal), Vitamin B₇ (Biotin), Vitamin B₉ (Folic acid, folinic acid), Vitamin B₁₂ (Cyanocobalamin, hydroxycobalamin, methylcobalamin), Vitamin C (Ascorbic acid), Vitamin D (Ergocalciferol, cholecalciferol), Vitamin E (Tocopherols, tocotrienols) and Vitamin K (phylloquinone, menaquinones). Vitamin precursors are also included.

Examples of salts are components comprising inorganic ions such as bicarbonate, calcium, chloride, magnesium, phosphate, potassium, and sodium or trace elements such as Co, Cu, F, Fe, Mn, Mo, Ni, Se, Si, Ni, Bi, V and Zn. Examples are copper(II) sulphate pentahydrate (CuSO₄·5H₂O), sodium chloride (NaCl), calcium chloride (CaCl₂·2H₂O), potassium chloride (KCI), iron(II) sulphate, ferric ammonium citrate (FAC), sodium phosphate monobasic anhydrous (NaH₂PO₄), magnesium sulphate anhydrous (MgSO₄), sodium phosphate dibasic anhydrous (Na₂HPO₄), magnesium chloride hexahydrate (MgCl₂·6H₂O), zinc sulphate heptahydrate.

Examples of buffers are CO₂/HCO₃ (carbonate), phosphate, HEPES, PIPES, ACES, BES, TES, MOPS, and TRIS.

Examples of cofactors are thiamine derivatives, biotin, vitamin C, NAD/NADP, cobalamin, flavin mononucleotide and derivatives, glutathione, heme nucleotide phosphates, and derivatives.

Nucleic acid components, according to the present invention, are the nucleobases, like cytosine, guanine, adenine, thymine, or uracil, the nucleosides like cytidine, uridine, adenosine, guanosine and thymidine, and the nucleotides like adenosine monophosphate or adenosine diphosphate or adenosine triphosphate.

Feed media may have a different composition compared to full media. They typically comprise amino acids, trace elements, and vitamins. They might also comprise saccharide components but sometimes for production reasons the saccharide components are added in a separate feed.

Many biopharmaceutical production platforms are based on fed-batch cell culture protocols. The aim typically is to develop high-titer cell culture processes to meet increasing market demands and reduce manufacturing costs. Beside the use of high-performing recombinant cell lines, improvements in cell culture media and process parameters are required to realize the maximum production potentials.

In a fed-batch process, a basal medium supports initial growth and production, and a feed medium prevents the depletion of nutrients and sustains the production phase. The media are chosen to accommodate the distinct metabolic requirements during different production phases. Process parameter settings - including feeding strategy and control parameters - define the chemical and physical environments suitable for cell growth and protein production.

In a perfusion process, the cell culture medium is continuously added and removed from the bioreactors through pumps while the cells are retained in the bioreactor through a cell retention device. Advantages of perfusion are the possibility to reach very high cell densities (due to the constant medium exchange) and the possibility to produce very fragile recombinant proteins since the product can be removed from the bioreactor every day thus reducing the exposure time of the recombinant protein to high temperatures, oxidizing redox potentials or released cellular enzymes.

A process for perfusion cell culture typically comprises culturing cells in a bioreactor system comprising a bioreactor with a media inlet and a harvest outlet whereby
i. continuously or one or several times, preferably continuously, during the cell culture process new cell culture medium is inserted into the bioreactor via the media inlet
ii. continuously or one or several times during the cell culture process, preferably continuously, the harvest is removed from the bioreactor via the harvest outlet. The harvest typically comprises the target product produced by the cells, cells and liquid cell culture medium.

Poloxamers are amphiphilic polymers, with two hydrophilic blocks and a hydrophobic block in the middle. A poloxamer is a polyethylene glycol (PEG)/polypropylene glycol (PPG) tri-block copolymer whereby one PPG block is flanked on both sides with a PEG block. The polyethylene glycol (PEG) part is often also called polyethylene oxide (PEO) part. The polypropylene glycol (PPG) part is often also called the polypropylene oxide (PPO) part.

The poloxamer, CAS number 9003-11-6, which is typically used in cell culture applications is called poloxamer 188 and has the general formula I 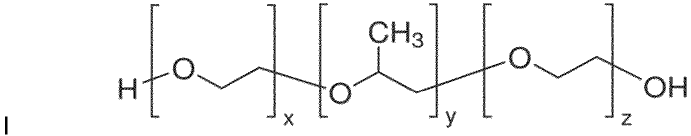 with x and z preferably independently being 75 to 85 and y preferably being 25 to 30. The poloxamers of the present invention can be found in the region with x = z = 3 to 72 and y = 5 to 12.

Further information about poloxamers can be found in Hagers Handbuch der Pharmazeutischen Praxis, volume 9 "Stoffe P-Z", 1994, pages 282 to 284.

The meroxapols have a reverse sequence compared to poloxamers. That is the reason why they are commonly called "reverse poloxamers". The central block of meroxapols is the PEO (polyethylene oxide), flanked on both sides with PPO (polypropylene oxide) blocks (Schmolka 1977).

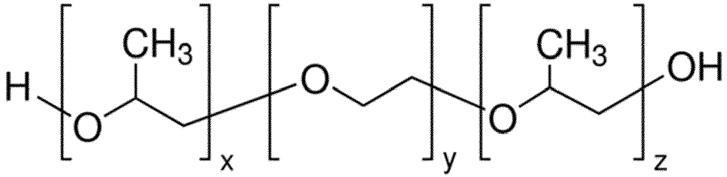

The meroxapols reported in the present invention can be found in the region with x=z=1 to 31 and y=13 to 173.

The poloxamer and meroxapols have the same CAS number 9003-11-6.

The nomenclature of poloxamer and meroxapols is similar and it is coded in the abovementioned review of Schmolka (Schmolka 1977). The first digits, multiplied by 100, refer to the molecular weight of the PPO block, and the last digit, multiplied per 10, gives the %EO of that molecule. To distinguish the meroxapols, frequently between the first and the last digits a R is added, to point out their reverse structure.

Some poloxamers and meroxapols are commercially available. Pluronic^{®} or Lutrol^{®} for poloxamers (e.g., a Pluronic^{®} solution, gel, or solid, such as Pluronic^{®} F-68) or Pluronic^{®} R for meroxapols.

Alternatively, poloxamers and meroxapols can be synthesized from raw materials according to methods known in the art (see, for example, U. S. Patent Nos. 3,036,118 and 3,740,421).

Table 1 summarizes exemplary meroxapols.

All listed meroxapols are characterized by determining their percentage of polyethylene oxide (%EO, w/w) and their molecular weight distribution. %EO was determined using ¹H NMR. The molecular weight distribution was determined with size exclusion chromatography (see Examples, Method 1). The peak molecular weight (Mp) was used to characterize each polymer.

| **Meroxapols** | **%EO** | **Mₚ (g/mol)** | **MW PPO (g/mol)** |
|---|---|---|---|
| Meroxapol 3R9 | 90.2 | 2893 | 284 |
| Meroxapol 5R9 | 89.8 | 4942 | 504 |
| Meroxapol 12R8 | 83.8 | 7629 | 1236 |
| Meroxapol 9R8 | 83.6 | 5476 | 898 |
| Meroxapol 3R8 | 83.1 | 2069 | 350 |
| Meroxapol 2R8 | 75.5 | 1020 | 250 |
| Meroxapol 8R7 | 74.5 | 3251 | 829 |
| Meroxapol 8R6 | 60.1 | 1985 | 792 |

Table 2 shows the same data for Poloxamer 188 which is used as reference in the Examples.

| **Poloxamer** | **%EO** | **Mₚ (g/mol)** | **MW PPO (g/mol)** |
|---|---|---|---|
| Poloxamer 188 | 82.3 | 8645 | 1530 |

### Detailed description of the invention

The gist of the present invention is the finding that certain meroxapols show improved properties for use in cell culture. They do not only provide shear protection like Poloxamer 188 but also show reduced foam formation. Suitable meroxapols are also non-toxic for the cells that are cultured.

Poloxamer 188 is often added to a cell culture to prevent hydrodynamic stress caused by sparging and/or agitation. It reduces surface tension but also typically increases foam formation. As cells tend to adhere to the surface of bubbles, they rise with the bubbles to the surface area, get trapped in the foam layer, and die. It has now been found that using meroxapols with a different composition, the positive shear stress protecting properties can be kept but in addition, the foam formation is reduced.

Consequently, the amount of an antifoam agent to reduce foam formation, like antifoam C, can be reduced or even preferably omitted when using the cell culture media according to the present invention. In a preferred embodiment, the media according to the present invention contain less, preferably at least 25% (w/w) less, more preferred less than half of the amount of an antifoam agent like antifoam C most preferred no antifoam agent that is used in an equivalent cell culture under the same conditions and with the same medium except that it does not comprise a meroxapol as defined in the present invention but poloxamer 188. A typical antifoam agent consists of an oil (hydrocarbon or poly(dimethylsiloxane)), dispersed hydrophobic solid particles, or a mixture of both. Examples of antifoam agents are preferably agents comprising polydimethylsiloxane (PDMS) as well as optionally silica particles, also called e.g., dimethicone or simethicone, like Dow-Corning Q7-2587 (O/W Emulsion with 30,4% PDMS, 1.2-2.1% SiO₂ particles), Antifoam C from Sigma Aldrich (O/W Emulsion 29.4% PDMS, 1.2-2-1% SiO₂ particles) and Foam Away from Gibco (30% Simethicone emulsion in water).

Preferably, the cell culture media according to the present invention or used in the present invention do not comprise poloxamer 188.

The meroxapols that have been identified to be especially suitable are meroxapols with a peak molecular weight Mₚ between 1000 and 8000 g/mol, preferably between 1000 and 5000 g/mol, and a polyethylene oxide percentage between 55 and 95% (w/w).

In a preferred embodiment, the peak molecular weight Mₚ is between 1800 and 3500 g/mol.

In another preferred embodiment, the PEO percentage is between 55 and 80%.

Very preferred, the meroxapols have a peak molecular weight Mₚ between 1800 and 3500 g/mol and the PEO percentage is between 55 and 80%.

A person skilled in the art knows how to use meroxapols as ingredients in cell culture media. He is aware of the suitable amount and format to use. Typically, the meroxapol is applied to the cell culture as part of the cell culture medium. But it can also be applied separately.

The meroxapol is typically present in form of either a solid, e.g., particles, or a liquid, e.g., oil or highly viscous liquid behaving like a paste, or an aqueous solution.

The cell culture medium according to the present invention is a cell culture medium comprising a meroxapol with a peak molecular weight Mₚ between 1000 and 8000 g/mol and an ethylene oxide percentage between 55 and 95% (w/w).

In a preferred embodiment, the meroxapols have a peak molecular weight Mₚ between 1800 and 3500 g/mol and the PEO percentage is between 55 and 80%.

In another preferred embodiment, the cell culture medium does not comprise an antifoam agent or comprises a reduced amount of antifoam agent, preferably less than 50% compared to a cell culture that does not comprise the meroxapols of the present invention but poloxamer 188. Preferably the medium also does not comprise poloxamer 188.

The concentration of the meroxapols in the cell culture medium is preferably between 0.1 to 10 g/L calculated for the liquid medium. In a very preferred embodiment, the amount of meroxapol is between 0.5 to 5 g/L calculated for the liquid medium. The meroxapol can be one type of meroxapol as defined above or a mixture of two or more of the meroxapols as defined above.

Preferably the cell culture medium is a dry powder or dry granulated medium or a liquid medium. In case of a dry medium, the medium is dissolved in a suitable amount of water or an aqueous buffer prior to use.

The cell culture medium of the present invention can be used for any type of cell culture. A cell culture is any setup in which cells are cultured.

A cell culture can be performed in any container suitable for the culture of cells, such as a petri dish, contact plate, bottle, tube, well, vessel, bag, flask, and/or tank. Preferably, it is performed in a bioreactor. Typically, the container is sterilized prior to use. Culturing is typically performed by incubation of the cells in an aqueous cell culture medium under suitable conditions such as suitable temperature, osmolality, aeration, agitation, etc. which limit contamination with foreign microorganisms from the environment. A person skilled in the art is aware of suitable incubation conditions for supporting or maintaining the growth/culturing of cells.

The present invention is thus also directed to a process for culturing cells whereby the cells are cultured in a liquid medium comprising a meroxapol with a peak molecular weight Mₚ between 1000 and 8000 g/mol and an ethylene oxide percentage between 55 and 95% (w/w).

The cell culture can be any set up suitable for culturing cells. Preferably it is a batch, a fed-batch, or a perfusion cell culture.

Preferably the process for culturing cells comprises the following steps:
a) providing a bioreactor
b) mixing the cells to be cultured with a cell culture medium according to the present invention.
c) incubating the mixture of step b).

In a preferred embodiment, the process does not involve the addition of an antifoam agent.

In one embodiment the process comprises the following steps:
a) providing a bioreactor
b) mixing the cells to be cultured with a cell culture medium according to the present invention
c) incubating the mixture of step b)
whereby a cell culture medium, which is, in this case, a feed medium, is added to the bioreactor, continuously over the whole time or once or several times within the cells incubation time of step c).

The feed medium may be a cell culture medium according to the present invention, but it may also be a feed medium that does not comprise a meroxapol. Preferably it does not comprise a meroxapol.

In one embodiment, the bioreactor is a perfusion bioreactor. A perfusion bioreactor is a bioreactor in which perfusion cell culture can be performed. It comprises the bioreactor vessel, which is typically closed during cell culture, a stirrer in the vessel, a line for introducing fresh medium, a harvest line for removing the harvest stream comprising cells, liquid medium and target product from the bioreactor and a cell retention device in the harvest line that retains the cells while the liquid part of the harvest can be collected. A review about perfusion cell culture providing details about favorable set ups can be found in "Perfusion mammalian cell culture for recombinant protein manufacturing - A critical review" Jean-Marc Bielser et al., Biotechnology Advances 36 (2018) 1328-1340.

In a perfusion process, the cell culture medium is continuously added and removed from the bioreactors through pumps while the cells are retained in the bioreactor through a cell retention device. Advantages of perfusion is the possibility to reach very high cell densities (due to the constant medium exchange) and the possibility to produce very fragile recombinant proteins since the product can be removed from the bioreactor every day thus reducing the exposure time of the recombinant protein to high temperatures, oxidizing redox potentials or released cellular proteases.

In one embodiment, the process of the present invention comprises culturing cells in a bioreactor system comprising a bioreactor with a media inlet and a harvest outlet whereby
i. continuously or one or several times, preferably continuously, during the cell culture process new cell culture medium according to the present invention is inserted into the bioreactor via the media inlet
ii. continuously or one or several times during the cell culture process, preferably continuously, harvest is removed from the bioreactor via the harvest outlet. The harvest typically comprises the target product produced by the cells, cells and liquid cell culture medium.

When using a cell culture medium according to the present invention the culture shows equal performance and reduced foam formation compared to a cell culture that is performed under the same conditions but with a medium comprising poloxamer 188 instead of the meroxapols as defined above.

The present invention is therefore also directed to a method for reducing foam formation in agitated and/or sparged cell cultures whereby the cells are cultured in a liquid medium comprising a meroxapol with a peak molecular weight Mₚ between 1000 and 8000 g/mol, preferably between 1000 and 5000 g/mol, and an ethylene oxide percentage between 55 and 95% (w/w) and the foam formation is reduced compared to cells cultured under the same conditions in a cell culture medium that comprises poloxamer 188 instead of a meroxapol with a peak molecular weight Mₚ between 1000 and 8000 g/mol, preferably between 1000 and 5000 g/mol, and an ethylene oxide percentage between 55 and 95% (w/w). Preferably no antifoam agent is used in the method for reducing foam formation.

The present invention is also directed to a method for culturing cells in agitated and/or sparged cell cultures whereby the cells are cultured in a liquid medium comprising a meroxapol with a peak molecular weight Mₚ between 1000 and 8000 g/mol, preferably between 1000 and 5000 g/mol, and an ethylene oxide percentage between 55 and 95% (w/w) and the amount of an antifoam agent in the liquid medium is reduced compared to cells cultured under the same conditions in a cell culture medium that comprises poloxamer 188 instead of a meroxapol with a peak molecular weight Mₚ between 1000 and 8000 g/mol, preferably between 1000 and 5000 g/mol, and an ethylene oxide percentage between 55 and 95% (w/w). Preferably the amount of antifoam agent is reduced by at least 25%, preferably by at least 50%. Most preferred no antifoam agent is used in the method for culturing cells in agitated and/or sparged cell cultures.

The media and methods of the present invention for the first time provide the possibility to, on the one hand provide shear stress protection, and on the other hand avoid the typically related complications of foam stabilization and the resulting requirement of adding an antifoam agent. Shear stress protection is provided without negative impact on foam formation and stabilization. It is even possible to increase the amount of meroxapol in the cell culture medium without a negative effect on foam formation. This provides more flexibility for the cell culture expert to set up ideal cultivation conditions.

Those results could not be expected. Poloxamer 188 and meroxapol 12R8 for example have a similar peak molecular weight (Mp), %EO, molecular weight of the PPO block, and values of surface tension of the solutions in cell culture media, but nevertheless they display a very different foam behavior. As can be seen from Example 2, meroxapol 12R8 solution resulted in a foam that did not reach the top of the column of the foam analyzer, and it eventually equilibrated to a plateau height between 5 and 6 cm. In contrast to that, the sample with poloxamer 188 reached the maximum foam height of the analyzer column within a very short time.

### Examples

### Method 1

All size exclusion chromatography (SEC) measurements are carried out as follows:
Calibration standards: PEG (Mp: 430, 982, 1960, 3020, 6690, 12300, 26100 and 44 000 g/mol)
Eluent: THF
Flow rate: 1 mL/min
Injection volume: 100 µL
Column: particle size = 5 µm, material = styrene-divinylbenzene
Temperature: 40 °C
Detector: refractive index (RI)

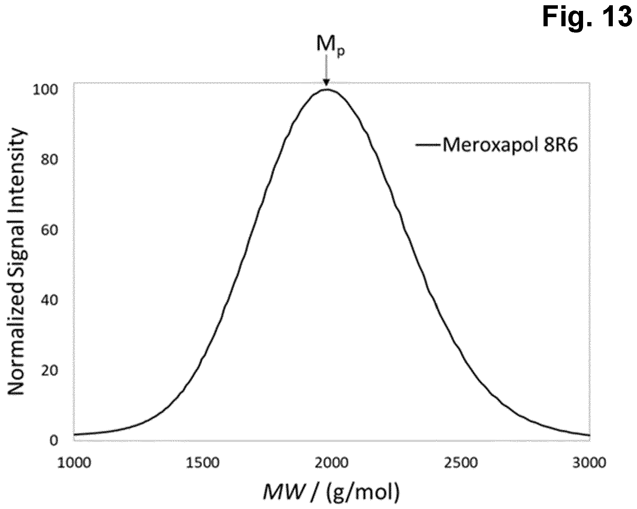

Molecular weight distribution curves were measured for all meroxapols and poloxamer 188 and Mp (peak maximum) was determined (Table 1 and 2). An exemplary distribution curve is shown in Figure 13, which shows the molecular weight distribution determined with size exclusion chromatography for meroxapol 8R6. Mp indicates the peak molecular weight of the specific meroxapol.

### Method 2:

Foam Evolution measurements of meroxapol solutions in cell culture media at 37°C

### Preparation of the meroxapol solutions in cell culture media

A solution of the target meroxapol in the cell culture media Cellvento^{®} 4CHO without poloxamer 188 (Merck KGaA) was prepared at the selected concentration and then stirred or mixed via a roller mixer for at least 2 hours until complete dispersion. The solutions were prepared by weight. The cell culture media was prepared according to the manufacturer's instructions.

### Foam measurements

The foam evolution was tested by using the Dynamic Foam Analyzer DFA 100 (Krüss GmbH).

The following parameters were adopted:
Temperature: 37 °C
Foaming method: Flow control (air)
Flow rate: 0.3 L/min
Liquid volume: 15 mL
Column: CY4575 - 40 mm t. prism
Filter: FL4551 - paper, 12-25 µm, ø 32 mm
Height illumination: Blue - λ = 469 nm
Sample holder: SH4511 - sparging
Camera height: 60 mm
Camera position: 2
Structure illumination: 30 %
Height illumination: 20 %

The solution was poured into the glass column of the Foam Analyzer DFA 100. The solution was heated till 37 °C because this is the temperature employed in bioreactors.

The gas flow was started, and the foam formation (foam height, liquid height, and foam structure) was recorded by the Foam Analyzer.

In case the foam reached the maximum column height (210 mm), the gas flow automatically stopped to prevent overflow and the foam decay without gas sparging was monitored for a selected time. In case the foam did not reach the top of the column, the gas continued flowing for the selected time (generally 60 min), then stopped, and the foam decay was monitored.

### Method 3

### Static surface tension measurement

The static surface tension was determined by using a Force Tensiometer K100C (Krüss GmbH). The Wilhelmy plate was employed for the measurement.

The following parameters were adopted.
Measurement temperature: 37 °C
Measurement time: 300 s
Data points: 200
Frequency: 1 Hz

The data are acquired until the standard deviation of the surface tension measured is less than 0.07 mN/m from its average value for 200 data points.

The solution was poured in the glass vessel of the force tensiometer K100C. The solution was heated till 37 °C because this is the temperature employed in bioreactors. Just before starting the measurement, the Wilhelmy plate was flamed until glowing red to eliminate contaminations and assure a contact angle equal to zero.

### Method 4

### Fed-batch cell culture in spin tubes

Fed-batch cell culture was performed to evaluate the protective performances of the meroxapols during cultivation. CHOK1 GS cells were employed. The culture was carried out in spin tubes (TPP, Art. No. 87050) with a final volume of 30 mL at 37 °C, 5% CO₂ humidified atmosphere, and 320 rpm rotation speed (incubator deflection 25 mm).

Before the start of the fed-batch, the cells were cultivated in the cell culture media Cellvento^{®} 4CHO (Merck KGaA, 103795) containing 2 g/L poloxamer 188. To this media HT-supplements 100x (sodium-hypoxanthine (10 mM) and thymidine (1,6 mM), Gibco^{®}, Life technologies, Art. No. 11067-030) and L-Methionine sulfoximine (Sigma Aldrich M5379) were added. For each condition, the cell culture media Cellvento^{®} 4CHO without poloxamer 188 (Merck KGaA) was employed and the selected meroxapols were added to achieve the concentration of 1 g/L. The solutions were stirred for at least two hours to allow complete dispersion.

On day 0 of the fed-batch, the cells were centrifuged for 5 min at 2000 rpm, then resuspended in 4CHO+HT without poloxamer 188 and pooled.

The CHOK1 GS cells were seeded in different poloxamer conditions (4 replicates each) with 0.2·10⁶ cells/mL.

The cells were fed with feed solution.

Glucose concentration was measured daily starting from D3. A continuous feeding of glucose on demand was maintained during the whole fermentation process.

The cultivation was carried out for 17 days.

Analysis of VCD and Viability with the ViCell (Beckman Coulter) and determination of IgG by using the Cedex (Bio HT Analyzer, Roche) were performed daily.

### Fed-batch cell culture in bioreactor

Fed-batch cell culture was performed in bioreactor to evaluate the protective and low foaming performances of the meroxapols during the cultivation of CHOK1 GS cells.

Glass bioreactors (Eppendorf, DASGIP^{®} Parallel bioreactor system Catalog. No. 76DG08CC and 76DG04CCBB) were used for the fed batch experiment. The bioreactors were equipped with a L-Sparger and a pitched blade impeller.

Foam traps were added to prevent a foam overflow.

Before the start of the fed-batch, the cells were cultivated and adapted respectively in each of the meroxapols and poloxamer 188 solutions in cell culture media Cellvento^{®} 4CHO (without poloxamer in the formulation, Merck KGaA, 4.74000.9999). The concentration was 2 g/L. To this media HT-supplements 100x (sodium-hypoxanthine (10 mM) and thymidine (1,6 mM), Gibco^{®}, Life technologies, Art. No. 11067-030) and L-Methionine sulfoximine (Sigma Aldrich M5379) were added

The fed batch was performed according to the following parameters.

| *T* / *°*C | pH | DO (%) | Agitation / RPM |
|---|---|---|---|
| 36.7 | 6.9 ± 0.2 | 50 | 280 |

0.2-0.4 % antifoam C was added only when the foam started to be collected in the foam trap. This event happened only for Poloxamer 188. With all the other meroxapols tested no significant foam formation occurred, therefore no antifoam C had to be added.

### Example 1 Foam analysis

The foaming behavior of the selected meroxapols was investigated. The experimental setup and the procedure are described in Method 2.

The foam stabilized by the meroxapols was compared with the foam stabilized by poloxamer 188, the benchmark for animal-free cell culture media. Different solutions were prepared. The meroxapols were added to Cellvento^{®} 4CHO cell culture media (without any poloxamer 188 present) at concentrations of 1000 ppm (1 g/L) and 2000 ppm (2 g/L).

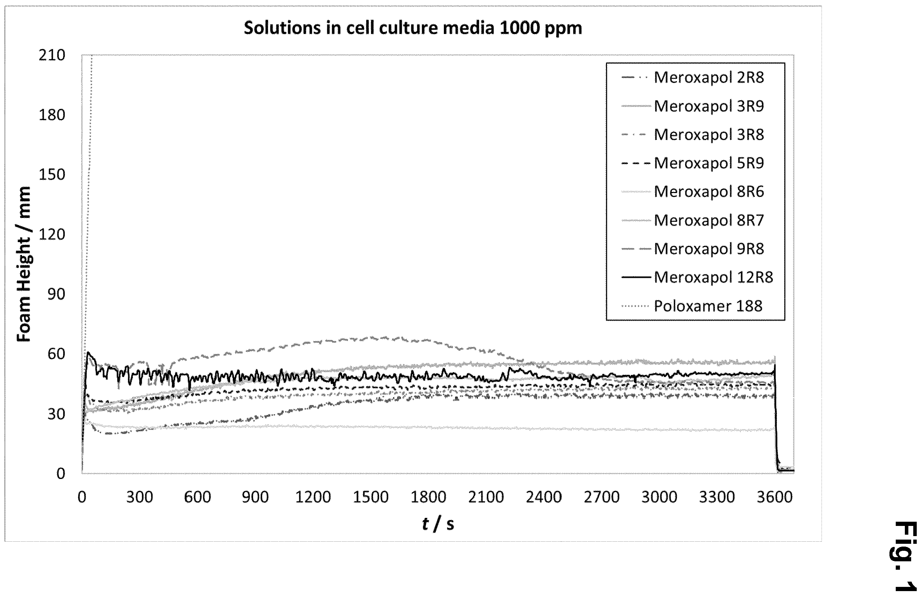

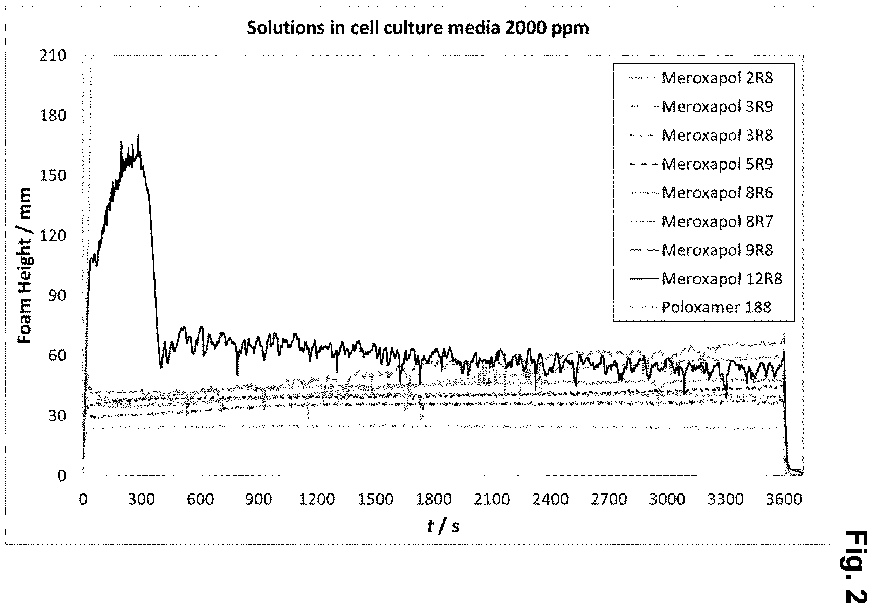

The foam evolution plots are reported in Figure 1 for the media with meroxapol concentration of 1000 ppm, and Figure 2 for media with meroxapol concentrations of 2000 ppm.

The foam stabilized by poloxamer 188 at both concentrations reached the maximum foam height of 210 mm (i.e., length of the foam analyzer column) within 50 s. At that point, the measurement was automatically stopped by the instrument to prevent overflow.

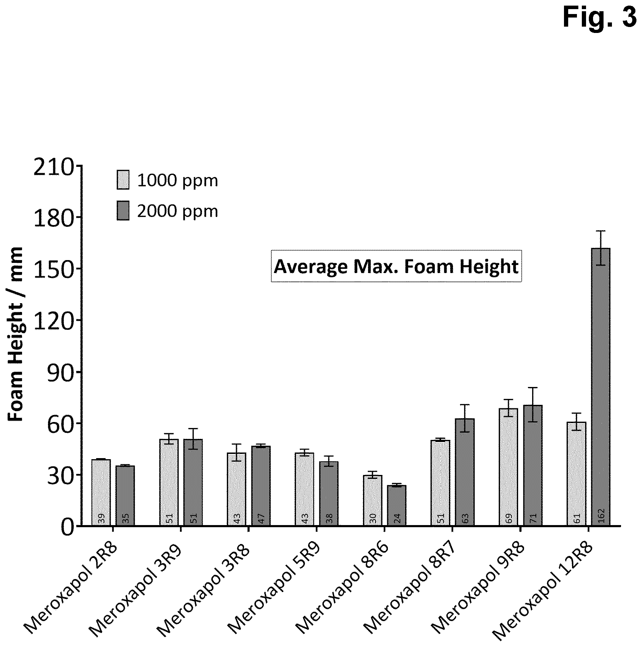

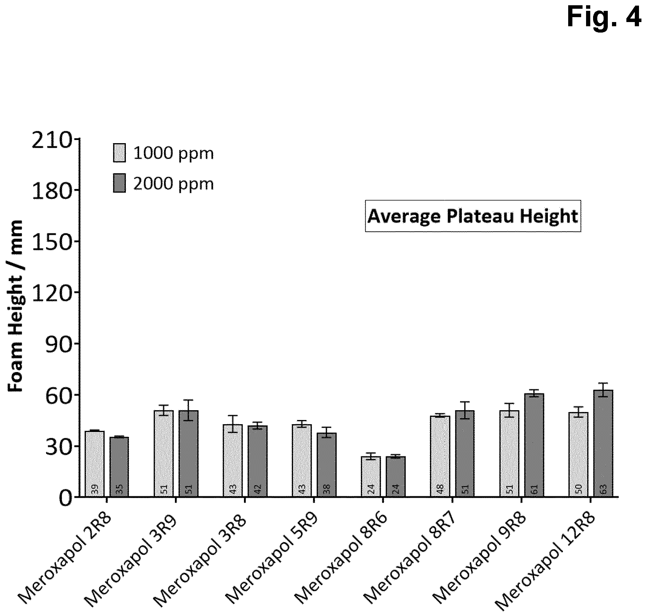

The meroxapols show a significantly different behavior. The foam stabilized by them did not reach the top of the column of the foam analyzer and the sparging of air continued for the whole measurement time. The foam grew rapidly within the first seconds of gas sparging, reaching a maximum height between 30 and 69 mm for the 1000 ppm solutions and between 24 and 162 mm for the 2000 ppm solutions. Then the foam height decreased quickly, and it stabilized to a constant height between 24 and 51 mm for 1000 ppm solutions and between 24 and 63 mm for the 2000 ppm solutions (see Figure 1, 2, 3, and 4).

The decrease of the foam height and the formation of a constant foam level, namely plateau height, can be explained in terms of an equilibrium between foam formation due to sparging and foam decay due to drainage and bubble coalescence.

Even though the meroxapols are less prone to stabilize foam compared to poloxamer 188, significant differences on the foam stabilization ability could be observed for the different meroxapols. Meroxapol 9R8 and 12R8 were more likely to stabilize foam and resulted in higher foam heights compared to the other meroxapols. Especially, meroxapol 8R6 displayed at both concentrations the lowest maximum and plateau foam heights.

Poloxamer 188 and meroxapol 9R8 have a very different foam behavior. The foam stabilized by poloxamer 188 reaches the maximum foam height of 21 cm in less than one minute, instead the foam of meroxapol 9R8 has a maximum foam height about 7 cm at both concentrations and reaches an equilibrium around 5 cm plateau height at 1000 ppm and 6 cm at 2000 ppm.

Another surprising observation is that, doubling the concentration, the average maximum foam height and plateau height remained similar. Only for meroxapol 12R8 the average maximum foam height was noticeably higher at 2000 ppm compared to the one at 1000 ppm.

These unexpected foam analysis data could be explained by the static surface tension measurements performed at 37 °C with the same solutions, just before the foam analysis. The concentration increase did not lead to a great decrease in surface tension, a difference between 1 and 2.5 mN/m. The biggest difference in surface tension with double concentration was measured for the meroxapol 12R8 solution (i.e., 4 mN/M), the same sample that resulted also in the biggest difference in average foam height.

No significant correlation has been found between surface tension values and average plateau height or average maximum foam height of each sample. In principle, a lower surface tension should lead to the stabilization of a higher interfacial area, hence to more bubbles and more foam. However, meroxapol 8R6 solution has a lower surface tension compared to the other samples (at 1000 ppm (48.7±0.2) mN/m and at 2000 ppm (46.5±0.3) mN/m), but it results in the lowest foam height.

Poloxamer 188 and meroxapol 12R8 have similar peak molecular weight (Mp), %EO, molecular weight of the PPO block, and values of surface tension of the solutions in cell culture media, but they display a very different foam behavior. Meroxapol 12R8 solution resulted in a foam that did not reach the top of the column of the foam analyzer, and it eventually equilibrated to a plateau height between 5 and 6 cm.

**Table 3 shows the static surface tension of meroxapol solutions in cell culture media at 1000 and 2000 ppm concentration. The data reported are the average with standard deviation of replicated measurements at 37 °C**

| | **1000 ppm** | | **2000 ppm** | |
|---|---|---|---|---|
| **Sample** | **Static surface tension (mN/m)** | **St. dev.** | **Static surface tension (mN/m)** | **St. dev.** |
| Meroxapol 2R8 | 54.19 | 0.04 | 52.0 | 0.3 |
| Meroxapol 3R9 | 55.20 | 0.02 | 54.5 | 0.1 |
| Meroxapol 3R8 | 54.7 | 0.4 | 53.63 | 0.02 |
| Meroxapol 5R9 | 53.6 | 0.6 | 52.5 | 0.1 |
| Meroxapol 8R6 | 48.7 | 0.2 | 46.5 | 0.3 |
| Meroxapol 8R7 | 49.9 | 0.3 | 48.5 | 0.4 |
| Meroxapol 9R8 | 50.9 | 0.3 | 49.4 | 0.2 |
| Meroxapol 12R8 | 46.8 | 0.8 | 43.0 | 0.2 |
| Poloxamer 188 | 45.6 | 0.1 | 44.3 | 0.6 |
| Cellvento^{®} 4CHO cell culture media without poloxamer added | 55.7 | 0.2 | 55.7 | 0.2 |

### Example 2:

### Meroxapols employed in fed-batch cultivation

### Spin tube cultivation

Fed-batch cell culture (see Method 4) was performed to investigate the shear stress protection properties of the different meroxapols compared to the benchmark poloxamer 188.

As observed in the foam analysis data, the meroxapols resulted in a much lower foam height compared to poloxamer 188 and they would be very appealing for cell culture if they were shear-stress protectors.

The fed-batch culture of CHOK1 GS cells was carried out for 17 days in spin tubes, according to the procedure described in Method 4. All meroxapols were tested in Cellvento^{®} 4CHO cell culture media not containing any poloxamer 188. Each solution had concentration of 1 g/L meroxapol (i.e., 1000 ppm).

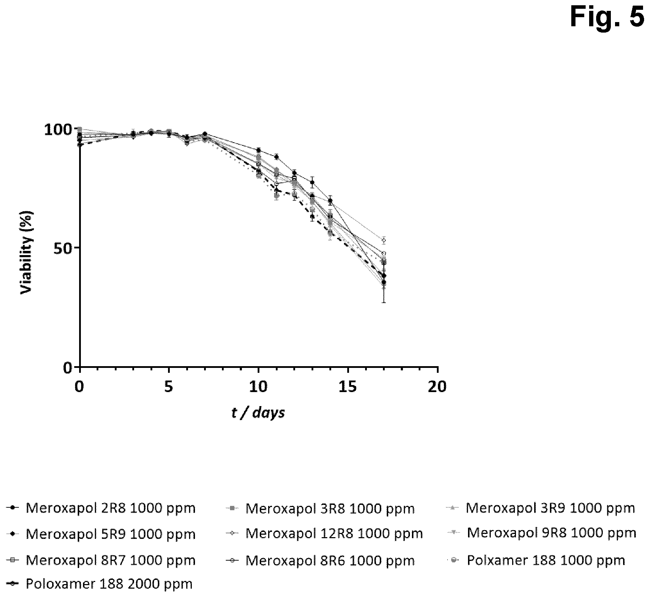

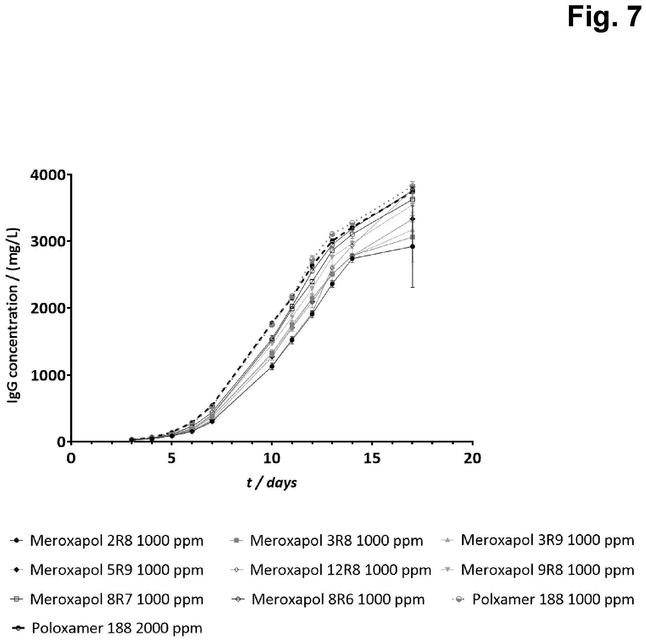

As shown in Figure 5, 6, and 7, the viability, viable cell density and IgG productivity of the cells cultivated in the meroxapols are all comparable with the data of the cells cultivated in poloxamer 188, even with double concentration of 188 (2 g/L).

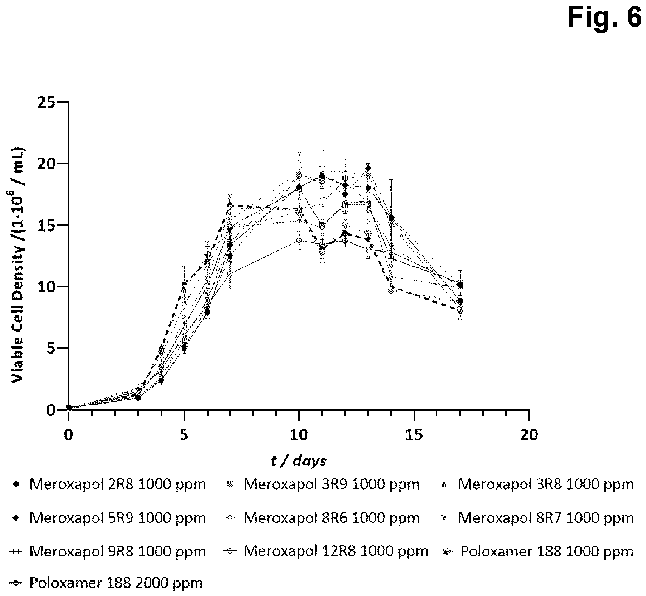

Figure 6 points out in a clearer way the differences among the different samples. Until day 7, all the samples have comparable VCDs within their error range. After day 10 of culture, the cells cultivated in in poloxamer 188 at both concentrations start to decrease, instead the cells cultivated in the other meroxapols continue to grow till day 13.

The antibody production of cells cultivated in meroxapols, especially 8R7 and 8R6, is comparable to cells cultivated in poloxamer 188.

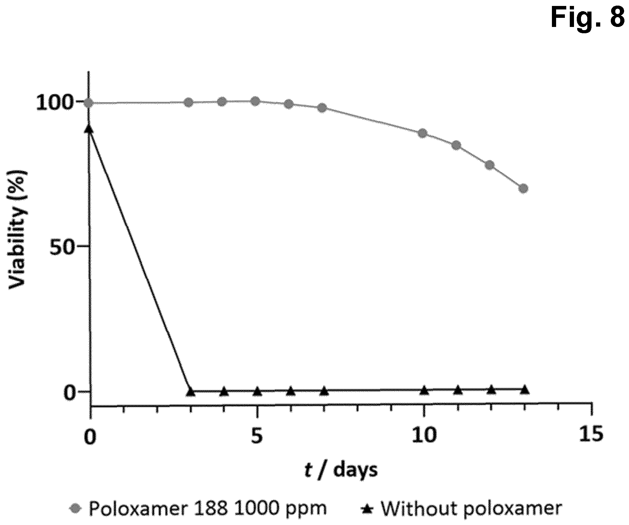

This experiment shows that the described meroxapols are effective shear-stress protector for fed-batch cultivation in spin tubes and the antibody productivity is comparable to the cultivation with poloxamer 188. It is remarkable to observe how well the meroxapols support the cell growth, especially considering how essential poloxamer 188 is for the cultivation, even in conditions of low shear stress, like spin-tube fed batch. The crucial role of poloxamer 188 is pointed out in Figure 8, where the cell viability during a cultivation in absence of any poloxamer is compared with the condition including 1 g/L poloxamer 188. Either Poloxamer 188 or a low-foaming alternative like the meroxapols described in this invention are necessary to achieve cell growth in suspension cultivation.

The cultivation in spin tubes does not allow to compare the foam formation of the different conditions due to its setup (no sparging present, therefore no foam formed). The evaluation of the shear stress protection combined with low foaming abilities will be performed with the bioreactor cultivation.

### Bioreactor cultivation

Fed-batch cell culture in bioreactor was performed to evaluate at the same time the shear stress protection and the foam stabilization properties of the different meroxapols compared to the benchmark poloxamer 188. The cultivation in bioreactor, in contrast to the spin tube culture, involves air sparging and mechanical stirring, which could lead to foam formation. Therefore, bioreactors are suitable to evaluate the performance of meroxapols.

The fed-batch culture of CHOK1 GS cells was carried out for 14 days in glass bioreactors, according to the procedure described in Method 4. All meroxapols were tested in Cellvento^{®} 4CHO cell culture media not containing any poloxamer 188. Each condition had a concentration of 2 g/L (i.e., 2000 ppm) meroxapol or poloxamer 188.

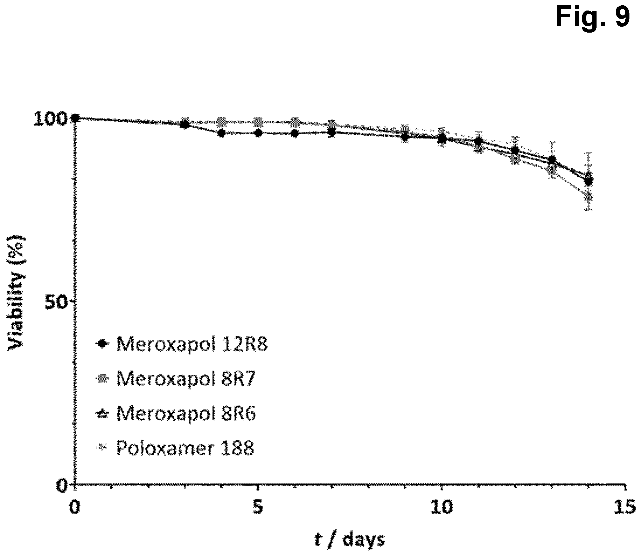

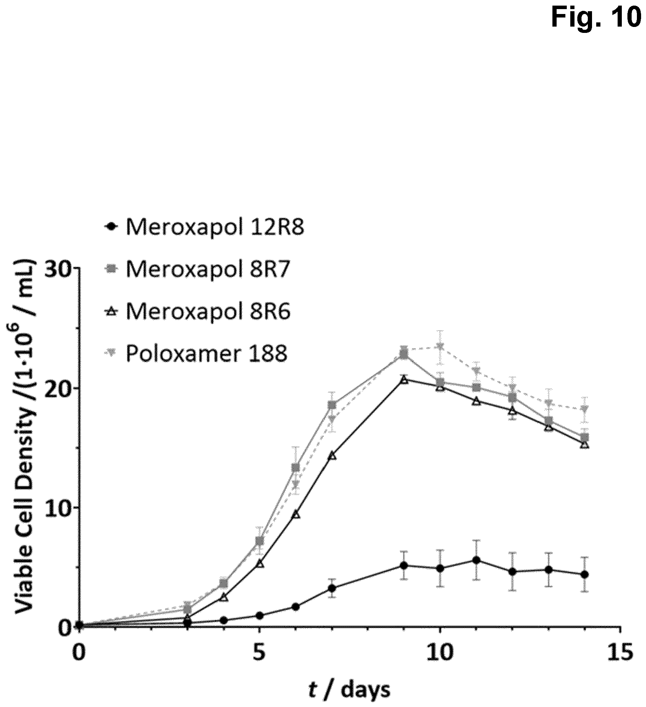

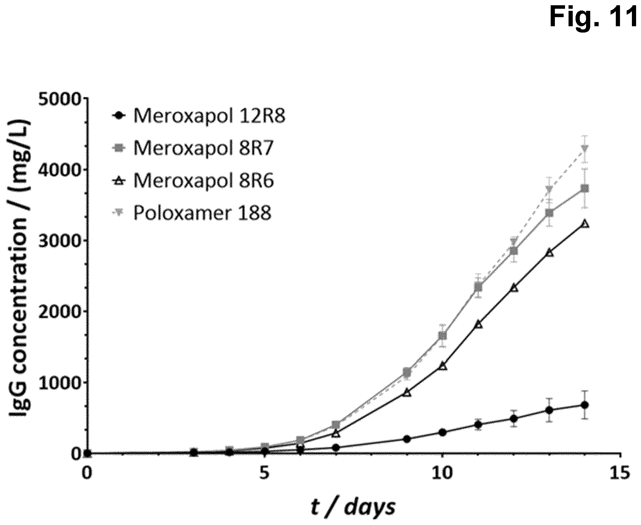

As shown in Figure 9, 10, and 11, the viability, viable cell density and IgG productivity of the cells cultivated in the meroxapols 8R7 and 8R6 are comparable with the data of the cells cultivated in poloxamer 188. The cells cultivated in meroxapol 12R8 display a lower VCD and IgG titer, but comparable viability. This observation can be a hint that meroxapol 12R8 is a less efficient shear stress protector compared to the other conditions. This cultivation setup with an increased shear stress allowed us to evaluate deeper the protection performances and differentiate the meroxapols.

The antibody production of cells cultivated in meroxapols 8R7 and 8R6 is comparable to cells cultivated in poloxamer 188.

The difference between the foam stabilized by the meroxapols and poloxamer 188 is striking. The foam formed in the bioreactors containing the meroxapols is very little over the whole cultivation. The maximum foam level was registered at the end of the culture on day 14, 150 mL for meroxapol 8R7, 50 mL for meroxapol 8R6 and none for meroxapol 12R8. Instead poloxamer 188 reached a foam level of 800 mL after only 7 days and antifoam C was needed to avoid foam overflow. Without the addition of antifoam C, the overflow could have led to overpressure, leakages, contaminations, and eventually to the abortion of the experiments. Starting from day 10, antifoam C was added every day to the three bioreactors containing poloxamer 188. Nevertheless, the foam was stabilized more and more. The cultivations supported by the meroxapols did not require any antifoam C and they did not present any risk of foam out.

It was demonstrated that the use of meroxapol 8R7 and 8R6 led to comparable cell growth and antibody productivity to poloxamer 188 and, at the same time, reduced greatly the foam formation avoiding the need of antifoam C. The employment of the selected meroxapols can be very useful for bioprocessing, reducing the risks and avoiding the issues related to the antifoam addition.

## Claims

1. A cell culture medium comprising a meroxapol with a peak molecular weight Mₚ between 1000 and 8000 g/mol and a polyethylene oxide percentage between 55 and 95% (w/w).

2. A cell culture medium according to claim 1, **characterized in that** the peak molecular weight Mₚ is between 1000 and 5000 g/mol.

3. A cell culture medium according to claim 1 or claim 2, **characterized in that** the polyethylene oxide percentage is between 55 and 80%.

4. A cell culture medium according to one or more of claims 1 to 3, **characterized in that** the cell culture medium comprises the meroxapol in an amount between 0.1 to 10 g/L calculated for the liquid medium.

5. A cell culture medium according to one or more of claims 1 to 4, **characterized in that** the cell culture medium is a chemically defined medium.

6. A cell culture medium according to one or more of claims 1 to 5, **characterized in that** the cell culture medium is a dry powder or a dry compacted medium.

7. A cell culture medium according to one or more of claims 1 to 6, **characterized in that** the cell culture medium comprises at least one or more saccharide components, one or more amino acids, one or more vitamins or vitamin precursors, one or more salts, one or more buffer components, one or more co-factors and one or more nucleic acid components.

8. A cell culture medium according to one or more of claims 1 to 7, **characterized in that** it does not comprise any antifoam agent.

9. A process for culturing cells whereby the cells are cultured in a liquid medium comprising a meroxapol with a peak molecular weight Mₚ between 1000 and 8000 g/mol and a polyethylene oxide percentage between 55 and 95% (w/w).

10. A process for culturing cells according to claim 9, **characterized in that** the process includes agitation and/or sparging.

11. A process for culturing cells according to claim 9 or 10, **characterized in that** the peak molecular weight Mₚ is between 1000 and 5000 g/mol.

12. A process for culturing cells according to one or more of claims 9 to 11, **characterized in that** the polyethylene oxide percentage is between 55 and 80%.

13. A process for culturing cells according to one or more of claims 9 to 12, **characterized in that** the amount of antifoam agent in the liquid medium is reduced compared to a cell culture medium that is otherwise identical but comprises poloxamer 188 instead of a meroxapol.

14. A process for culturing cells according to one or more of claims 9 to 13, **characterized in that** the liquid medium does not comprise any antifoam agent.

15. A method for reducing foam formation in agitated and/or sparged cell cultures whereby the cells are cultured in a liquid medium comprising a meroxapol with a peak molecular weight Mₚ between 1000 and 8000 g/mol and a polyethylene oxide percentage between 55 and 95% (w/w) and the foam formation is reduced compared to cells cultured under the same conditions in a cell culture medium that comprises poloxamer 188 instead of a meroxapol with a peak molecular weight Mₚ between 1000 and 8000 g/mol and a polyethylene oxide percentage between 55 and 95% (w/w).

## Patentansprüche

1. Zellkulturmedium, enthaltend ein Meroxapol mit einem Peak-Molekulargewicht Mₚ zwischen 1000 und 8000 g/mol und einem Polyethylenoxid-Prozentsatz zwischen 55 und 95 Gew.-%.

2. Zellkulturmedium nach Anspruch 1, **dadurch gekennzeichnet, dass** das Peak-Molekulargewicht Mₚ zwischen 1000 und 5000 g/mol liegt.

3. Zellkulturmedium nach Anspruch 1 oder Anspruch 2, **dadurch gekennzeichnet, dass** der Polyethylenoxid-Prozentsatz zwischen 55 und 80 % liegt.

4. Zellkulturmedium nach einem oder mehreren der Ansprüche 1 bis 3, **dadurch gekennzeichnet, dass** das Zellkulturmedium das Meroxapol in einer für das flüssige Medium berechneten Menge zwischen 0,1 und 10 g/l enthält.

5. Zellkulturmedium nach einem oder mehreren der Ansprüche 1 bis 4, **dadurch gekennzeichnet, dass** das Zellkulturmedium ein chemisch definiertes Medium ist.

6. Zellkulturmedium nach einem oder mehreren der Ansprüche 1 bis 5, **dadurch gekennzeichnet, dass** das Zellkulturmedium ein trockenes Pulver oder ein trockenes verdichtetes Medium ist.

7. Zellkulturmedium nach einem oder mehreren der Ansprüche 1 bis 6, **dadurch gekennzeichnet, dass** das Zellkulturmedium mindestens eine oder mehrere Saccharidkomponenten, eine oder mehrere Aminosäuren, ein(e) oder mehrere Vitamine oder Vitaminvorstufen, ein oder mehrere Salze, eine oder mehrere Pufferkomponenten, einen oder mehrere Co-Faktoren und eine oder mehrere Nukleinsäurekomponenten enthält.

8. Zellkulturmedium nach einem oder mehreren der Ansprüche 1 bis 7, **dadurch gekennzeichnet, dass** es kein Antischaummittel enthält.

9. Verfahren zum Kultivieren von Zellen, wobei die Zellen in einem flüssigen Medium kultiviert werden, das ein Meroxapol mit einem Peak-Molekulargewicht Mₚ zwischen 1000 und 8000 g/mol und einem Polyethylenoxid-Prozentsatz zwischen 55 und 95 Gew.-% enthält.

10. Verfahren zum Kultivieren von Zellen nach Anspruch 9, **dadurch gekennzeichnet, dass** das Verfahren Rühren und/oder Durchblasen beinhaltet.

11. Verfahren zum Kultivieren von Zellen nach Anspruch 9 oder 10, **dadurch gekennzeichnet, dass** das Peak-Molekulargewicht Mₚ zwischen 1000 und 5000 g/mol liegt.

12. Verfahren zum Kultivieren von Zellen nach einem oder mehreren der Ansprüche 9 bis 11, **dadurch gekennzeichnet, dass** der Polyethylenoxid-Prozentsatz zwischen 55 und 80 % liegt.

13. Verfahren zum Kultivieren von Zellen nach einem oder mehreren der Ansprüche 9 bis 12, **dadurch gekennzeichnet, dass** die Menge an Antischaummittel in dem flüssigen Medium im Vergleich zu einem Zellkulturmedium, das ansonsten identisch ist, aber Poloxamer 188 anstelle eines Meroxapols enthält, reduziert ist.

14. Verfahren zum Kultivieren von Zellen nach einem oder mehreren der Ansprüche 9 bis 13, **dadurch gekennzeichnet, dass** das flüssige Medium kein Antischaummittel enthält.

15. Verfahren zur Verringerung der Schaumbildung in gerührten und/oder durchblasenen Zellkulturen, wobei die Zellen in einem flüssigen Medium kultiviert werden, das ein Meroxapol mit einem Peak-Molekulargewicht Mₚ zwischen 1000 und 8000 g/mol und einem Polyethylenoxid-Prozentsatz zwischen 55 und 95 Gew.-% enthält, und die Schaumbildung im Vergleich zu Zellen, die unter denselben Bedingungen in einem Zellkulturmedium kultiviert werden, das Poloxamer 188 anstelle eines Meroxapols mit einem Peak-Molekulargewicht Mₚ zwischen 1000 und 8000 g/mol und einem Polyethylenoxid-Prozentsatz zwischen 55 und 95 Gew.-% enthält, reduziert ist.

## Revendications

1. Milieu de culture cellulaire comprenant un méroxapol ayant un poids moléculaire maximal Mₚ compris entre 1000 et 8000 g/mol et un pourcentage d'oxyde de polyéthylène compris entre 55 et 95% (p/p).

2. Milieu de culture cellulaire selon la revendication 1, **caractérisé en ce que** le poids moléculaire maximal Mₚ est compris entre 1000 et 5000 g/mol.

3. Milieu de culture cellulaire selon la revendication 1 ou la revendication 2, **caractérisé en ce que** le pourcentage d'oxyde de polyéthylène est compris entre 55 et 80%.

4. Milieu de culture cellulaire selon l'une ou plusieurs parmi les revendications 1 à 3, **caractérisé en ce que** le milieu de culture cellulaire comprend le méroxapol selon une quantité comprise entre 0,1 et 10 g/L calculée pour le milieu liquide.

5. Milieu de culture cellulaire selon l'une ou plusieurs parmi les revendications 1 à 4, **caractérisé en ce que** le milieu de culture cellulaire est un milieu chimiquement défini.

6. Milieu de culture cellulaire selon l'une ou plusieurs parmi les revendications 1 à 5, **caractérisé en ce que** le milieu de culture cellulaire est une poudre sèche ou un milieu sec compacté.

7. Milieu de culture cellulaire selon l'une ou plusieurs parmi les revendications 1 à 6, **caractérisé en ce que** le milieu de culture cellulaire comprend au moins un ou plusieurs composants saccharidiques, un ou plusieurs acides aminés, un(e) ou plusieurs vitamines ou précurseurs de vitamine, un ou plusieurs sels, un ou plusieurs composants de tampon, un ou plusieurs cofacteurs et un ou plusieurs composants d'acide nucléique.

8. Milieu de culture cellulaire selon l'une ou plusieurs parmi les revendications 1 à 7, **caractérisé en ce qu'**il ne comprend pas d'agent antimousse.

9. Procédé de culture de cellules, dans lequel les cellules sont cultivées dans un milieu liquide comprenant un méroxapol ayant un poids moléculaire maximal Mₚ compris entre 1000 et 8000 g/mol et un pourcentage d'oxyde de polyéthylène compris entre 55 et 95% (p/p).

10. Procédé de culture de cellules selon la revendication 9, **caractérisé en ce que** le procédé comporte une agitation et/ou un barbotage.

11. Procédé de culture de cellules selon la revendication 9 ou 10, **caractérisé en ce que** le poids moléculaire maximal Mₚ est compris entre 1000 et 5000 g/mol.

12. Procédé de culture de cellules selon l'une ou plusieurs parmi les revendications 9 à 11, **caractérisé en ce que** le pourcentage d'oxyde de polyéthylène est compris entre 55 et 80%.

13. Procédé de culture de cellules selon l'une ou plusieurs parmi les revendications 9 à 12, **caractérisé en ce que** la quantité d'agent antimousse dans le milieu liquide est réduite par rapport à un milieu de culture cellulaire qui est par ailleurs identique mais comprend un poloxamère 188 au lieu d'un méroxapol.

14. Procédé de culture de cellules selon l'une ou plusieurs parmi les revendications 9 à 13, **caractérisé en ce que** le milieu liquide ne comprend pas d'agent antimousse.

15. Méthode de réduction de la formation de mousse dans des cultures cellulaires sous agitation et/ou sous barbotage, dans laquelle les cellules sont cultivées dans un milieu liquide comprenant un méroxapol ayant un poids moléculaire maximal Mₚ compris entre 1000 et 8000 g/mol et un pourcentage d'oxyde de polyéthylène compris entre 55 et 95% (p/p) et la formation de mousse est réduite par rapport à des cellules cultivées dans les mêmes conditions dans un milieu de culture cellulaire qui comprend un poloxamère 188 au lieu d'un méroxapol ayant un poids moléculaire maximal Mₚ compris entre 1000 et 8000 g/mol et un pourcentage d'oxyde de polyéthylène compris entre 55 et 95% (p/p).
